(19) Europäisches Patentamt
European Patent Office
Office européen des brevets

(11) Publication number: **0 080 449**
**B1**

(12) **EUROPEAN PATENT SPECIFICATION**

(45) Date of publication of patent specification: **03.07.85**

(51) Int. Cl.[4]: **C 07 C 11/18,** C 07 C 1/253

(21) Application number: **82870057.5**

(22) Date of filing: **26.10.82**

(54) **Synthesis of isoprene from linear butenes.**

(30) Priority: **28.10.81 US 315803**

(43) Date of publication of application:
**01.06.83 Bulletin 83/22**

(45) Publication of the grant of the patent:
**03.07.85 Bulletin 85/27**

(84) Designated Contracting States:
**DE FR GB IT NL**

(56) References cited:
**WO-A-80/01690**
**DE-A-2 163 396**
**SU-A- 721 116**

**Chemical Abstracts vol. 61, no. 13, 21 December 1964, Columbus, Ohio, USA V. V. KAS'YANOV et al. "Kinetics of 1-butene isomerization with the double bond shift on alumina A-1", columns 15952h-15953b**

**The file contains technical information submitted after the application was filed and not included in this specification**

(73) Proprietor: **MONSANTO COMPANY**
**Patent Department 800 North Lindbergh Boulevard**
**St. Louis, Missouri 63166 (US)**

(72) Inventor: **Forster, Denis**
**32 Woodwest Drive**
**St. Louis Missouri 63124 (US)**
Inventor: **Barker, George Edward**
**1616 Red Gate**
**St. Louis Missouri 63141 (US)**

(74) Representative: **Lunt, John Cooper et al**
**Monsanto Europe S.A. Patent Department**
**Avenue de Tervuren 270-272 Letter Box No 1**
**B-1150 Brussels (BE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 080 449 B1**

**Description**

This invention relates to a method of making isoprene from linear butenes.

Isoprene is a key monomer for the manufacture of synthetic rubbers. It is primarily used to make cis-polyisoprene which is a stereospecific rubber having the same monomer as natural rubber. Four fundamental processes have been used commercially to construct the isoprene $C_5$ skeleton from smaller carbon units. Only two of these processes are still in commercial operation. One of the production routes involves condensing acetylene and acetone, followed by hydrogenation and dehydration:

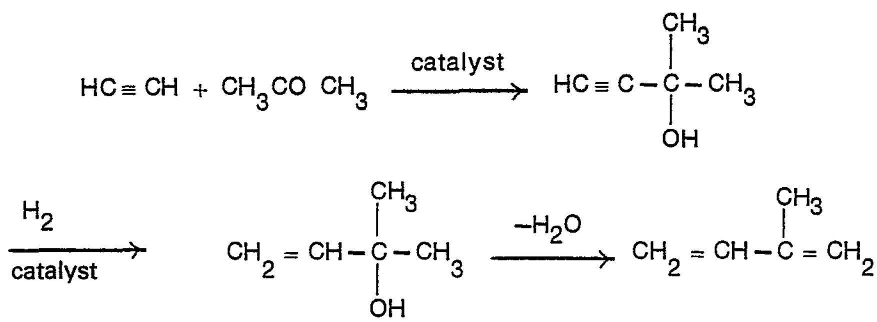


The other route involves, as a first step, the Prins reaction between formaldehyde and isobutylene.

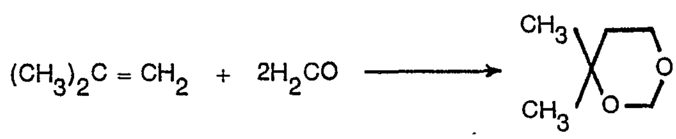

In a subsequent step the intermediate derivative is catalytically cracked at elevated temperatures.

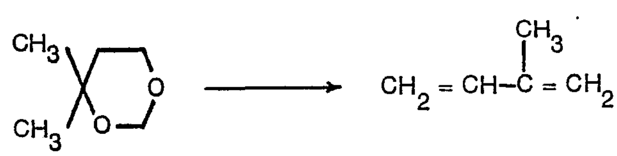

See, e.g., French Patent 1,294,716; *Chem. Absts. 57:* 15309.

It is generally recognized that the isobutylene-formaldehyde route represents an advance over the route using expensive acetylene feedstock. Nevertheless isobutylene also is a relatively expensive source of $C_4$ hydrocarbon, and formaldehyde is a far more expensive source of carbon than either methanol or carbon monoxide. Accordingly, a method of making isoprene from more economically available raw materials would have distinct advantages.

*Description of the Invention*

The invention described and claimed herein provides a method for the synthesis of isoprene using low cost linear butenes and synthesis gas ($CO/H_2$) as the raw materials.

The advent of large-scale production of methyl tertiary butyl ether (MTBE) as an octane booster, particularly for premium-grade unleaded gasoline, has given rise to the availability of large quantities of linear butenes as a raffinate from the process. This availability is due to the fact that the MTBE process is presently being practiced by reacting methanol with refinery $C_4$ streams containing a mixture of $C_4$ olefin and paraffin isomers. A typical composition of such a stream is shown in Table 1.

TABLE 1

| Component | Vol. % |
|---|---|
| Isobutene | 44—49 |
| 1-butene | 24—28 |
| 2-butene (cis & trans) | 19—21 |
| n-butane | 6—8 |
| isobutane | 2—3 |

In the MTBE process only the isobutene reacts, thereby leaving a mixture of linear butenes and paraffins as a raffinate. Since no large scale chemical uses presently exist for linear butenes, their value is currently set by their utility in fuel-related outlets, in particular as alkylate for gasoline.

In order to generate the required skeleton of isoprene from a linear $C_4$ olefin it is required that the additional carbon be added at the 2-position. It is known that the hydroformylation reaction (or oxo process) will attach a formyl group to an olefin double bond.

$$>C=C< + CO + H_2 \xrightarrow{\text{catalyst}} -\!>C-C<\!- CHO$$

According to this process, discovered by Roelin of Ruhrchemie A.G. (see U.S. Patent 2,327,066), alcohols are prepared from olefins, carbon monoxide and hydrogen in the liquid phase in the presence of metallic cobalt catalysts such as Raney cobalt or cobalt carbonyls at 115°—190°C and high pressures of 10132—20265 kPa (100—200 Atm.) in a Fischer-Tropsch type reaction. The process can be carried out in two stages in which the initial stage yields principally aldehydes, which can then be reduced to the alcohol.

In order to generate a molecule with the same backbone as isoprene, the above hydroformylation reaction must be stereochemically directed to the 2-position when conducted with linear butenes. In accordance with the present invention, this result is achieved by hydroformylating cis or trans butene-2 with a homogeneous rhodium catalyst in the presence of excess organic ligand. By contrast, cobalt catalysts of the prior art are unsuitable for this reaction since even with butene-2 as the feedstock the major aldehyde isomer obtained is the linear n-pentanal. It is known from U.S. Patent 3,965,192 that rhodium catalysts with excess ligand in the hydroformylation of butene-2 can produce the aldehydes in a 10:1 ratio of 2-methylbutanal to n-pentanal.

As Table 1 shows, the predominant linear butene isomer found in refinery $C_4$ streams is butene-1. The rhodium hydroformylation catalyst with excess ligand is known to give predominantly n-pentanal with this olefin isomer (see, e.g., U.S. Patents 3,917,661 and 3,946,082). However, cis and trans butene-2 are more stable, thermodynamically, than butene-1 and hence, in accordance with the present invention, isomerization of the mixed linear butenes is carried out to provide a mixture generally containing less than about 3% of butene-1. Thus, when the mixture of linear butenes is first isomerized and then subjected to hydroformylation reaction conditions using a rhodium catalyst with excess ligand present, the product is 2-methylbutanal.

In a third step of the present invention, 2-methylbutanal is dehydrated to isoprene using an acidic heterogeneous catalyst, i.e.,

$$CH_3-CH_2-\underset{}{\overset{CH_3}{\overset{|}{C}}}H-CHO \xrightarrow{\text{catalyst}} CH_2=CH-\overset{CH_3}{\overset{|}{C}}=CH_2 + H_2O$$

Thus the invention described and claimed herein provides a new and useful route to isoprene based on linear butenes and synthesis gas which utilizes 3 steps:

1) mixed linear butenes $\xrightarrow{\text{isomerization catalyst}}$ cis and trans butene-2

2) 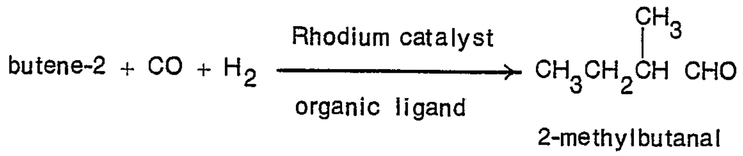


3) 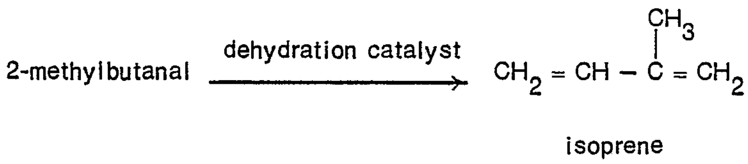


The initial isomerization of butene-1 to butene-2 is carried out in the presence of a double bond isomerization catalyst or a non-skeletal isomerization catalyst. By the term "non-skeletal" isomerization catalyst is meant a catalyst which does not cause any appreciable chain branching. Illustrative examples of suitable catalysts for this reaction step are alumina, aluminum sulfate, aluminum phosphate, phosphoric acid deposited on alumina, thoria, palladized asbestos and molybdenum sulfide as described in a treatise by F. Asinger, "Monoolefins Chemistry and Technology", Pergammon Press, 1968, at page 1021 and references cited therein. The isomerization preferably is carried out in the liquid phase at elevated temperature and pressure. Further background information on the isomerization of butene-1 to butene-2, including useful catalysts and reaction conditions, can be had by reference to a comprehensive review on olefin isomerization by H. N. Dunning, *Ind. Eng. Chem. 45,* 551—564 (1953). The catalysts and reaction conditions for said isomerization disclosed by Dunning can be used for the isomerization step herein. An alumina and Pd catalyst combination (see, e.g. U.S. Patent 4,179,581) is particularly useful in the isomerization of butene-1 to butene-2. See also Chemical Abstracts Vol. 61 (1964) No. 13, Columns 15952h—15953b.

Under a preferred set of conditions for the isomerization step, butene-1 is isomerized to a mixture of cis and trans butene-2 at about 80°C and about 20 to 8 kPa (300 psi — 20.4 Atm.) over a fixed bed of alumina catalyst with about 0.3% Pd. A small amount of hydrogen is used as a catalyst promoter in this reaction and the hydrogen is methanated to preclude catalyst poisoning by CO.

It will be appreciated that the linear butenes used in the isomerization reaction need not be separated from the accompanying paraffins in the $C_4$ refinery stream since only the isomerized butenes will react in the next step.

The resulting butene-2 mixture, or butene-2 enriched mixture, is then hydroformylated to 2-methyl butanal in the presence of a homogeneous rhodium catalyst and an excess of organic ligand. The rhodium catalyst should be soluble in the reaction medium along with the reactants. Illustrative examples of suitable rhodium catalysts for the hydroformylation step are rhodium salts such as rhodium sulfate, rhodium acetate, rhodium nitrate and, less desirably, rhodium halides such as rhodium trichloride and rhodium tribromide (see, e.g., U.S. Patent 4,110,404). Also suitable are rhodium carbonyl catalysts such as rhodium carbonyl chloride — $[Rh(CO)_2\ C1]_2$ (see, e.g., U.S. Patent 4,292,448); rhodium chelates such as rhodium acetylacetonate (see, e.g., U.S. Patent 4,110,404; rhodium catalysts complexed with phosphine such as Wilkinson's catalyst — chlorotris(triphenylphosphine)Rh—$RhCl(PPh_3)_3$ (see, e.g., U.S. Patent 3,946,082); and rhodium carbonyl complexes with phosphines such as carbonylhydrotris(triphenylphosphine)-Rh—$RhH(CO)(PPh_3)_3$ (see, e.g., U.S. Patents 3,965,192 and 4,200,591—592), and tricarbonylbis(triphenylphosphine)rhodium(1+) tetraphenylborate — $Rh(CO_3)(PPh_3)_2BPh_4$ (see e.g., U.S. Patent 4,052,461). Other suitable rhodium catalysts are the organic complexes of rhodium with organic molecules such as ethylene, propylene, 1,5-cycloöctadiene, 1,5-hexadiene and bicyclo-2.2.1-hepta-2,5-diene. Illustrative of such organic rhodium complexes is rhodium dicylo-1,5-octadiene (see, e.g., U.S. Patent 4,210,608); di-μ-chlorobis [(1,2,5,6-η)-1,5-cycloöctadiene) di-rhodium or dimeric cycloöctadienyl rhodium chloride (see, e.g., Ger. Offen, 2,051,319); and [(1,2,5,6-η)-1,5-cycloöctadiene] bis(triphenylphosphine)rhodium(1+) tetraphenylborate (see, e.g., U.S. Patent 4,052,461).

The preferred rhodium catalysts are non-halide containing rhodium compounds.

The organic ligand should be capable of binding to the rhodium ion and may be firmly bound as in the above rhodium complexes and/or may remain uncoordinated as a free compound. The tertiary organo phosphorus, arsenic and antimony compounds are particularly useful as such ligands for the rhodium ion. Illustrative examples of these ligands for use with the rhodium catalyst are the organic phosphines and phosphites (see, e.g., U.S. Patent 3,801,646). Especially useful are the trialkylated phosphines such as triethyl phosphine, tributylphosphine and triphenylphosphine and other such $C_3$—$C_{90}$ alkyl-, aryl- and alkaryl phosphines as well as the corresponding phosphites such as, e.g., triethylphosphite, tributylphosphite and triphenylphosphite. Organic arsines and stibines also can be used as the ligand but the stibines are less preferred than the corresponding phosphines and arsines.

An exemplary rhodium catalyst and organic ligand combination for use in the hydroformylation step of the present invention is a mixture of Rh(cycloöctadiene)acetylacetonate with an excess of triphenylphosphine. This catalyst/ligand combination has resulted in greater than 98% selectivity to 2-methylbutanal as described in a more detailed example hereinafter.

The catalyst generally is present in an amount of from $10^{-1}$ to $10^{-5}$ moles (Rh basis) per mole of butene-2 feed and the volume percent of $CO/H_2$ generally ranges from 10/90 to 90/10 $CO/H_2$ in the hydroformylation step herein.

Under preferred conditions of the hydroformylation step, the cis and trans butene-2 mixture is reacted with synthesis gas ($CO:H_2$ = 1:1) at a temperature of from 70°C to 150°C and a pressure of 101—10132 kPa (1—100 atmospheres) in the presence of 60 to 150 moles of triphenylphosphine per mole of rhodium catalyst (Rh basis). The hydroformylation reaction is exothermic and most of the heat of reaction can be removed by vaporizing the reactor effluent. Coarse reactor temperature control also can be achieved by adjusting the reactor pressure.

The 2-methybutanal product recovered from the hydroformylation step is then dehydrated to isoprene at elevated temperature in the presence of an acidic heterogeneous catalyst. Useful catalysts for this purpose are acid dehydration catalysts such as phosphoric acid anhydride, boric acid anhydride, acid silicon dioxides and acid titanium oxides, and other such acidic compounds, as described for example in DE—A—2163396.

A preferred catalyst for the dehydration step in this invention is boron phosphate which is described in British Patent 1,385,348, Example 8, (*Chem. Absts. 79:* 106002) and USSR Patent 721,116 (*Chem. Absts. 98:* 9348). According to the latter patent, the catalyst is prepared by mixing concentrated $H_3PO_4$ and boric acid, evaporating the mixture, grinding, calcining and then processing with a mixture of steam and air containing 15—75 vol % steam at 450—650°C for 1—8 hours.

Other particularly desirable catalyst materials for the dehydration step are silica-beads having surface areas ≦100m²/gm, such as those available commercially from PPG Industries, e.g., PPG Silica Beads Type M0—1, 1.59 mm, having the following characteristics:

Surface Area = 50—60m²/gm
Average Pore Diameter = 80 nm
Average Pore Volume = 0.6 cm³/gm

The dehydration reaction is endothermic and under preferred conditions the reaction is performed in the vapor phase over a fixed bed of the catalyst at elevated temperature of from 200°C to 400°C. The desired isoprene product is recovered in high yield, e.g. selectivity to isoprene as high as 85% at 80% conversion of the 2-methylbutanal as described in a more detailed example hereinafter.

The following examples will further illustrate the invention although it should be understood that the invention is not limited to these specific examples.

Example 1

A $C_4$ fed refinery stream having a component composition within the following approximate range of concentrations is used as a source of linear butenes.

TABLE 2

| Compound | Vol. % |
|---|---|
| Isobutene | 44—49 |
| butene-1 | 24—28 |
| butene-2 (cis and trans) | 19—21 |
| n-butane | 6—8 |
| isobutane | 2—3 |

The $C_4$ refinery stream is fed to the bottom of a butene isomerization reactor having a fixed bed of alumina catalyst with 0.3% Pd. A small amount of methanated hydrogen is fed to the top of the reactor as a catalyst promoter. The isomerization reaction takes place at 80°C and 2068 kPa (300 psi — 20.4 Atm). The resulting isomerized cis and trans butene-2 can be separated from the other $C_4$ components and recovered or can be used directly without being separated from admixture with the entrained paraffins before proceeding to the next step.

Butene-2 (cis and trans) which can be obtained from a refinery stream as above, or which is otherwise available as merchant butene-2, can be hydroformylated to 2-methyl butanal as follows:

In this example, merchant butene-2 (1732 gm) was charged to an autoclave together with Rh(cyclooctadiene) acetylacetonate (4.08 gm) and triphenylphosphine (170.37 gm). The autoclave was pressured to 6895 kPa gauge (1000 psig) with a 1:1 mixture of $CO:H_2$ and then heated to stirring to 110°C.

The reaction temperature was maintained for four hours. The autoclave was cooled and vented. The resulting product was analyzed by gas chromatography and shown to contain greater than 98% 2-methylbutanal.

2-methylbutanal (15% in helium) was passed over a solid crystalline boron phosphate catalyst (liquid hourly space velocity, 0.7 $hr^{-1}$) at 350°C. The gaseous product was analyzed by a gas chromatographic technique and it was found that the selectivity to isoprene was 85% at 80% conversion of the 2-methylbutanal.

Example 2

A $C_4$ fed refinery stream processed by a MTBE plant (which removes isobutylene) has the following composition:

TABLE 3

| Component | Mole % |
|---|---|
| n-butane | 7.5 |
| isobutane | 7.5 |
| isobutylene | 2.0 |
| butene-1 | 41.5 |
| cis butene-2 | 16.5 |
| trans butene-2 | 25.0 |

The refinery stream is fed to the isomerization reactor and the butene-2 enriched stream is recovered as in Example 1, above, without separation of the paraffins.

Butene-2 (cis and trans) from the isomerization reactor step is hydroformylated with CO and $H_2$ in a 1:1 ratio (synthesis gas) at 115°C and 807 kPa (117 psi — 8 Atm.) with a homogeneous rhodium catalyst and ligand as in Example 1. 60 moles of triphenylphosphine ligand per mole of Rh is used. Zinc oxide packing is used to remove traces of chloride from the synthesis gas feed and molecular sieve packing is used to remove traces of water from the butene-2 feed. The resulting 2-methylbutanal is separated from unreacted material and recovered.

2-methylbutanal from the hydroformylation reaction step is dehydrated to isoprene in an endothermic reaction at 400°C and 689 kPa (100 psi — 6.8 Atm.) over a fixed bed of silica beads. 10 moles of steam dilution per mole of the aldehyde are used. The resulting isoprene is separated from the reaction zone and recovered.

Various other examples will be apparent to the ordinary person skilled in the art after reading the present disclosure without departing from the spirit and scope of the invention and it is intended that all such further examples be included within the scope of the appended claims.

**Claims**

1. A method of synthesizing isoprene from linear butenes comprising

(a) isomerizing mixed linear butenes in the presence of a double bond isomerization catalyst to a mixture of cis and trans butene-2,

(b) hydroformylating said butene-2 mixture with CO and $H_2$ in the presence of homogeneous rhodium catalyst and an excess of organic ligand to 2-methylbutanal,

(c) and dehydrating said 2-methylbutanal at elevated temperature in the presence of acidic heterogeneous catalyst to isoprene.

2. The method of Claim 1 in which the isomerization catalyst is alumina.

3. The method of Claim 1 in which the isomerization is carried out in the liquid phase at elevated temperature of about 80°C and pressure of about 2068 kPa over a fixed bed of alumina catalyst with about 0.3% palladium.

4. The method of Claim 1 in which the homogeneous rhodium catalyst is Rh(cyclooctadiene)acetylacetonate and the organic ligand is triphenylphosphine.

5. The method of Claim 1 in which the hydroformylation is carried out with synthesis gas at a temperature of from 70°C to 150°C and a pressure of from 101 to 10132 kPa (1 to 100 atmospheres) in the presence of from 60 moles to 150 moles of triphenylphosphine per mole of rhodium catalyst.

6. The method of Claim 1 in which the acidic heterogeneous dehydration catalyst is boron phosphate.

7. The method of Claim 1 in which the acidic heterogeneous dehydration catalyst is silica beads.

8. The method of Claim 1 in which the dehydration is carried out over a fixed bed of catalyst in the vapor phase at a temperature of from 200°C to 400°C.

**Patentansprüche**

1. Verfahren zur Synthese von Isopren aus linearen Butenen, wobei

(a) gemischte lineare Butene in Gegenwart eines Doppelbindungs-Isomerisierungskatalysators zu einem Gemisch von cis- und trans-Buten-2 isomerisiert werden,

(b) das Buten-2-Gemisch mit CO und $H_2$ in Gegenwart eines homogenen Rhodiumkatalysators und einem Überschuß an organischem Liganden zu 2-Methylbutanal hydroformyliert wird,

(c) und das 2-Methylbutanal bei erhöhter Temperatur in Gegenwart von saurem heterogenem Katalysator zu Isopren dehydratisiert wird.

2. Verfahren nach Anspruch 1, worin der Isomerisierungskatalysator Aluminiumoxid ist.

3. Verfahren nach Anspruch 1, worin die Isomerisierung in der flüssigen Phase bei erhöhter Temperatur von etwa 80°C und einem Druck von etwa 2068 kPa über einem Festbett von Aluminiumoxid-Katalysator mit etwa 0,3% Palladium durchgeführt wird.

4. Verfahren nach Anspruch 1, worin der homogene Rhodiumkatalysator Rh(Cyclooctadien)acetylacetonat ist und der organische Ligand Triphenylphosphin ist.

5. Verfahren nach Anspruch 1, worin die Hydroformylierung mit Synthesegas bei einer Temperatur von 70°C bis 150°C und einem Druck von 101 bis 10132 kPa (1 bis 100 Atmosphären) in Gegenwart von 60 Mol bis 150 Mol Triphenylphosphin pro Mol Rhodiumkatalysator durchgeführt wird.

6. Verfahren nach Anspruch 1, worin der saure heterogene Dehydratisierungskatalysator Borphosphat ist.

7. Verfahren nach Anspruch 1, worin der saure heterogene Dehydratisierungskatalysator Siliciumdioxidperlen sind.

8. Verfahren nach Anspruch 1, worin die Dehydratisierung über einem Festbett von Katalysator in der Dampfphase bei einer Temperatur von 200°C bis 400°C durchgeführt wird.

**Revendications**

1. Procédé de synthèse d'isoprène à partir de butènes linéaires, caractérisé en ce qu'il comprend:

(a) l'isomérisation d'un mélange de butènes linéaires en présence d'un catalyseur d'isomérisation par déplacement de la double-liaison en un mélange de butène-2 cis et trans;

(b) l'hydroformylation du mélange de butène-2 cis et trans avec CO et $H_2$ en présence d'un catalyseur de rhodium homogène et d'un excès de ligand organique en 2-méthylbutanal;

(c) et la déshydratation du 2-méthylbutanal à température élevée en présence d'un catalyseur hétérogène acide en isoprène.

2. Procédé selon la revendication 1, caractérisé en ce que le catalyseur d'isomérisation est l'alumine.

3. Procédé selon la revendication 1, caractérisé en ce que l'on met en oeuvre l'isomérisation en phase liquide à une température élevée d'environ 80°C et sous une pression d'environ 2068 kPa sur un lit fixe de catalyseur d'alumine contenant environ 0,3% de palladium.

4. procédé selon la revendication 1, caractérisé en ce que le catalyseur de rhodium homogène est l'acétylacétonate de Rh(cyclooctadiène) et en ce que le ligand organique est la triphénylphosphine.

5. Procédé selon la revendication 1, caractérisé en ce qu'on met en oeuvre l'hydroformylation avec du gaz de synthèse à une température comprise entre 70°C et 150°C et sous une pression comprise entre 101 et 10 132 kPa (1 à 100 atmosphères) en présence de 60 moles à 150 moles de triphénylphoshine par mole de catalyseur de rhodium.

6. Procédé selon la revendication 1, caractérisé en ce que le catalyseur de déshydratation hétérogène acide est le phosphate de bore.

7. Procédé selon la revendication 1, caractérisé en ce que le catalyseur de déshydratation hétérogène acide est constitué par des perles de silice.

8. Procédé selon la revendication 1, caractérisé en ce que l'on met en oeuvre la déshydratation sur un lit fixe de catalyseur en phase vapeur à une température comprise entre 200°C et 400°C.